# EUROPEAN PATENT APPLICATION

(11) **EP 4 245 250 A2**
(43) Date of publication of application: **20.09.2023**
(21) Application number: 23168355.8
(22) Date of filing: 15.08.2017
(51) Int. Cl.: A61B 90/50

(54) **SURGICAL NAVIGATION SYSTEM FOR PROVIDING AN AUGMENTED REALITY IMAGE DURING OPERATION**

(62) Divisional of application: 17186306.1
(71) Applicant: Holo Surgical Inc., Deerfield, IL 60015 (US)
(72) Inventor: Siemionow, Krzysztof B., Chicago, 60610 (US); Luciano, Cristian J., Evergreen Park, 60805 (US)
(74) Representative: Kancelaria Eupatent.pl Sp. z.o.o

(57) **Abstract**

A surgical navigation system comprising: a tracking system (125) comprising means for real-time tracking of at least one of: a surgeon's head (106), a 3D display (142), a patient anatomy (105) and surgical instruments (107) to provide current position and/or orientation data; a source of at least one of: an operative plan (161, 162), a patient anatomy data (163) and a virtual surgical instrument model (164); a surgical navigation image generator (131) configured to generate a surgical navigation image (142A) comprising at least one of: the patient anatomy data (163), operative plan data (161, 162) and virtual surgical instruments (164), in accordance to the current position and/or orientation data provided by the tracking system (125); wherein the 3D display (142) is configured to emit the surgical navigation image (142A) towards a see-through mirror (141) that is partially transparent and partially reflective, such that an augmented reality image (141A) collocated with the patient anatomy in the surgical field (108) underneath the see-through mirror (141) is visible to a viewer looking from above the see-through mirror (141) towards the surgical field (108).

## Description

### TECHNICAL FIELD

The present disclosure relates to surgical navigation systems, in particular to a system and method for operative planning and execution of a medical procedure.

### BACKGROUND

Some of typical functions of a computer-assisted surgery (CAS) system with navigation include presurgical planning of a procedure and presenting preoperative diagnostic information and images in useful formats. The CAS system presents status information about a procedure as it takes place in real time, displaying the preoperative plan along with intraoperative data. The CAS system may be used for procedures in traditional operating rooms, interventional radiology suites, mobile operating rooms or outpatient clinics. The procedure may be any medical procedure, whether surgical or non-surgical.

Surgical navigation systems are used to display the position and orientation of surgical instruments and medical implants with respect to presurgical or intraoperative medical imagery datasets of a patient. These images include pre and intraoperative images, such as two-dimensional (2D) fluoroscopic images and three-dimensional (3D) magnetic resonance imaging (MRI) or computed tomography (CT).

Navigation systems locate markers attached or fixed to an object, such as surgical instruments and patient. Most commonly these tracking systems are optical and electromagnetic. Optical tracking systems have one or more stationary cameras that observes passive reflective markers or active infrared LEDs attached to the tracked instruments or the patient. Eye-tracking solutions are specialized optical tracking systems that measure gaze and eye motion relative to a user's head. Electro-magnetic systems have a stationary field generator that emits an electromagnetic field that is sensed by coils integrated into tracked medical tools and surgical instruments.

Incorporating image segmentation processes that automatically identify various bone landmarks, based on their density, can increase planning accuracy. One such bone landmark is the spinal pedicle, which is made up of dense cortical bone making its identification utilizing image segmentation easier. The pedicle is used as an anchor point for various types of medical implants. Achieving proper implant placement in the pedicle is heavily dependent on the trajectory selected for implant placement. Ideal trajectory is identified by surgeon based on review of advanced imaging (e.g., CT or MRI), goals of the surgical procedure, bone density, presence or absence of deformity, anomaly, prior surgery, and other factors. The surgeon then selects the appropriate trajectory for each spinal level. Proper trajectory generally involves placing an appropriately sized implant in the center of a pedicle. Ideal trajectories are also critical for placement of inter-vertebral biomechanical devices.

Another example is placement of electrodes in the thalamus for the treatment of functional disorders, such as Parkinson's. The most important determinant of success in patients undergoing deep brain stimulation surgery is the optimal placement of the electrode. Proper trajectory is defined based on preoperative imaging (such as MRI or CT) and allows for proper electrode positioning.

Another example is minimally invasive replacement of prosthetic/biologic mitral valve in for the treatment of mitral valve disorders, such as mitral valve stenosis or regurgitation. The most important determinant of success in patients undergoing minimally invasive mitral valve surgery is the optimal placement of the three dimensional valve.

The fundamental limitation of surgical navigation systems is that they provide restricted means of communicating to the surgeon. Currently-available navigation systems present some drawbacks.

Typically, one or several computer monitors are placed at some distance away from the surgical field. They require the surgeon to focus the visual attention away from the surgical field to see the monitors across the operating room. This results in a disruption of surgical workflow. Moreover, the monitors of current navigation systems are limited to displaying multiple slices through three-dimensional diagnostic image datasets, which are difficult to interpret for complex 3D anatomy.

The fact that the screen of the surgical navigation system is located away from the region of interest (ROI) of the surgical field requires the surgeon to continuously look back and forth between the screen and the ROI. This task is not intuitive and results in a disruption to surgical workflow and decreases planning accuracy.

For example, a system of such type is disclosed in a US patent US9532848, which discloses a system for assisting a user manipulating an object during a surgery, the system comprising a tracking device for tracking the object and for generating tracking data for the object and a sterilized displaying device located in a volume within the sterile field defined as being above a plane of and delimited by an operating table and below the shoulders of an operator standing next to a patient lying on the operating table, the displaying device being supported directly by the operating table. Even though the displaying device is positioned very close to the patient being operated, the surgeon still needs to look back and forth between the screen and the ROI.

When defining and later executing an operative plan, the surgeon interacts with the navigation system via a keyboard and mouse, touchscreen, voice commands, control pendant, foot pedals, haptic devices, and tracked surgical instruments. Based on the complexity of the 3D anatomy, it can be difficult to simultaneously position and orient the instrument in the 3D surgical field only based on the information displayed on the monitors of the navigation system. Similarly, when aligning a tracked instrument with an operative plan, it is difficult to control the 3D position and orientation of the instrument with respect to the patient anatomy. This can result in an unacceptable degree of error in the preoperative plan that will translate to poor surgical outcome.

There is therefore a need to improve the surgical navigation systems to eliminate the above-mentioned drawbacks.

A GB patent application GB2536650 discloses a system and method for a near-eye display comprising at least one each of an optical combiner with a selective shutter layer at one side, a scene capturing device, a micro projector to project the captured video and a processor to determine at least one region of interest selected by a user and controlling the shutter layer to reduce the amount of light transferrable via a portion of the optical combiner corresponding to the determined region.

A US patent application US20170042631 discloses an intra-operative medical image viewing system comprising a display positionable between a surgeon and the patient during surgery, said display being configured to exhibit images to the surgeon overlaid on the patient; an image control unit configured to retrieve at least one image file from an image source and control said display to exhibit and modify at least a portion of the at least one image; and a plurality of peripheral devices, each configured to receive an image control input from the surgeon and in response to generate an image control signal in a respective user-interface modality. In one embodiment, the display is a wearable device, such as three-dimensional glasses. In another embodiment, the display is a see-through screen that is mounted on a non-wearable frame that is fixed, as seen in the embodiment figure, closer to the patient than to the surgeon's head. The display is capable of locating a medical image between the eyes of the surgeon and the patient. The system can fuse together multiple images, such as pre-operative and/or intra-operative images to conform to the surgeon's visual perspective.

The above-mentioned solutions partially eliminate the drawback of typical prior art surgical navigation systems wherein the computer monitors are placed at some distance away from the operative field and require the surgeon to focus the visual attention away from the surgical field to see the monitors across the operating room.

However, one drawback related to head-worn apparatus, such as three-dimensional display glasses disclosed in the above-mentioned publications, is that they must provide high-resolution display to generate a signal of satisfactory quality, in particular for a large field of view. Moreover, the head-worn displays have a limited field of view. Moreover, they need very precise positioning control, as the slightest changes of surgeon's head require repositioning of the displayed image - it's quite hard to precisely and consistently keep the head-mounted display at exactly the same position and orientation with respect to the surgeon's eyes, perfect virtual/real collocation of patient anatomy is difficult to achieve. Augmented reality head-mounted devices are designed to display virtual images located at roughly the same height as the user's head, and at a certain distance from the user's body, which is not appropriate for surgery. This makes almost impossible to achieve virtual/real anatomical superposition at a location that is reachable by the surgeon's hands. Also, the significant weight of the head-worn apparatus or head mounted display (HMD) can produce tiredness on the surgeon after a long period of usage. The surgeon has limited range of movements, it makes it harder to maneuver the head during the surgery and talk to other physicians. Moreover, use of head mounted displays involves vergenceaccommodation conflict, that causes fatigue and discomfort of the user.

These drawbacks can be avoided partially by providing a fixed non-wearable see-through screen, that may have a larger display area and therefore high-resolution image is easier to be displayed therein. However, the frame as disclosed in US20170042631, being located closer to the patient than to the surgeon's head, may obstruct the freedom of instrument and hand movement during the operating procedure.

There is, therefore, a need to further improve the surgical navigation systems to eliminate the above-mentioned drawbacks.

### SUMMARY

There is disclosed herein a surgical navigation system comprising: a tracking system comprising means for real-time tracking of at least one of: a surgeon's head, a 3D display, a patient anatomy and surgical instruments to provide current position and/or orientation data; a source of at least one of: an operative plan, a patient anatomy data and a virtual surgical instrument model; a surgical navigation image generator configured to generate a surgical navigation image comprising at least one of: the patient anatomy data, operative plan data and virtual surgical instruments, in accordance to the current position and/or orientation data provided by the tracking system; wherein the 3D display is configured to emit the surgical navigation image towards a see-through mirror that is partially transparent and partially reflective, such that an augmented reality image collocated with the patient anatomy in the surgical field underneath the see-through mirror is visible to a viewer looking from above the see-through mirror towards the surgical field.

The 3D display may comprise a 3D projector for projecting the surgical navigation image towards a plurality of opaque mirrors for reflecting the surgical navigation image towards a projection screen for showing the surgical navigation image for emission towards the see-through mirror.

The 3D display may comprise a 3D monitor for showing the surgical navigation image for emission towards the see-through mirror.

The 3D display may comprise a 3D projector for projecting the surgical navigation image towards a projection screen for showing the surgical navigation image for emission towards the see-through mirror.

The see-through mirror can be configured to be positioned, when the system is in use, at a distance from the surgeon's head which is shorter than the distance from the surgical field of the patient anatomy.

The see-through mirror can be fixed to the 3D display by means of a detachable arm.

The see-through mirror can be fixed to the 3D display by means of a foldable arm.

The see-through mirror can be disposable.

The surgical navigation image generator can be controllable by an input interface comprising at least one of: foot-operable pedals, a microphone, a joystick, an eye-tracker.

The 3D display can be attached to an adjustable ceiling-mounted surgical boom in the operating room.

The 3D display can be attached to an adjustable floor-supported structure in the operating room.

The tracking system may comprise plurality of arranged fiducial markers, including a head array, a display array, a patient anatomy array, an instrument array, as well as a fiducial marker tracker, which is configured to determine in real time the position and orientation of each of the components of the surgical navigation system.

At least one of the head array, the display array, the patient anatomy array, the instrument array may contain several fiducial markers that are not all coplanar.

There is also disclosed a method for providing an augmented reality image during an operation, comprising: providing a tracking system comprising means for real-time tracing tracking of at least one of: a surgeon's head, a 3D display, a patient anatomy and surgical instruments to provide current position and/or orientation data; providing a source of at least one of: an operative plan, a patient anatomy data and a virtual surgical instrument model; generating, by a surgical navigation image generator, a surgical navigation image comprising at least one of: patient anatomy data, operative plan data and virtual surgical instruments, in accordance to the current position and/or orientation data provided by the tracking system; emitting the surgical navigation image towards a see-through mirror that is partially transparent and partially reflective, such that an augmented reality image collocated with the patient anatomy in the surgical field underneath the see-through mirror is visible to a viewer looking from above the see-through mirror towards the surgical field.

The intended use of this invention is both presurgical planning of ideal surgical instrument trajectory and placement, and intraoperative surgical guidance, with the objective of helping to improve surgical outcomes.

A combination of a navigated probe and a computer-assisted medical system is used for interactive creation of a trajectory for positioning of a medical device. A navigated probe facilitates the positioning of the medical device. The navigated probe is part of a computer-assisted medical system consisting of a 6-degree-of-freedom (DOF) tracker (optical, electromagnetic, inertial, or any other tracking technology) and a navigated structure. The navigated structure contains a graphical user interface (GUI) for displaying patient anatomy in three dimensions (3D), as well as a virtual representation of actual implanted medical devices (IMDs) and instruments during a surgical procedure in a real time.

The surgeon can control the navigated probe by looking at its virtual representation on the GUI and lining it up to the virtual representation of the organ to achieve a proper trajectory for medical implant placement. During the planning process, a virtual instrument is displayed on a 3D display device to indicate the dynamic 3D position and orientation of the medical device. The surgeon can interact with the probe and the computer-assisted medical system by either using a 6-DOF tracking device and/or by pressing on a set of pre-programed pedals or using other input interfaces, such as a microphone (for voice commands), a joystick, an eye-tracker (for gaze tracking).

The presented system and method solve the critical problems of typical surgical navigation systems. First, they allow the surgeon to focus the visual attention to the surgical field by superimposing 3D patient anatomy, surgical guidance, and orthogonal planes directly onto the area of patient anatomy where the surgery is performed, without requiring the surgeon to look away from the surgical field. Secondly, they provide the surgeon a more intuitive mechanism to define and execute an operative plan by simply handling the surgical instruments in a 3D workspace that perfectly matches the operative field, without requiring the surgeon to perform a disorienting mental mapping of the information displayed by the navigation system to the 3D position and orientation of the surgical instruments with respect to the complex 3D anatomy. Moreover, by using the presented system and method, the time of operation can be reduced, as the more intuitive communication means do not distract the surgeon and do not require additional time to look away from the ROI.

### BRIEF DESCRIPTION OF FIGURES

The surgical navigation system and method are presented herein by means of nonlimiting example embodiments shown in a drawing, wherein:
Figs. 1A-1C show a layout of a surgical room employing the surgical navigation system;
Figs. 2A, 2B show components of the surgical navigation system;
Figs. 3A, 3B, 3C, 3D, 3E show examples of augmented reality display;
Figs. 4A, 4B show an embodiment of a 3D display system comprising a projector.
Figs. 5A, 5B show eye tracking.
Figs. 6A, 6B, 6C, 6D, 6E show a foldable see-through mirror.

### DETAILED DESCRIPTION

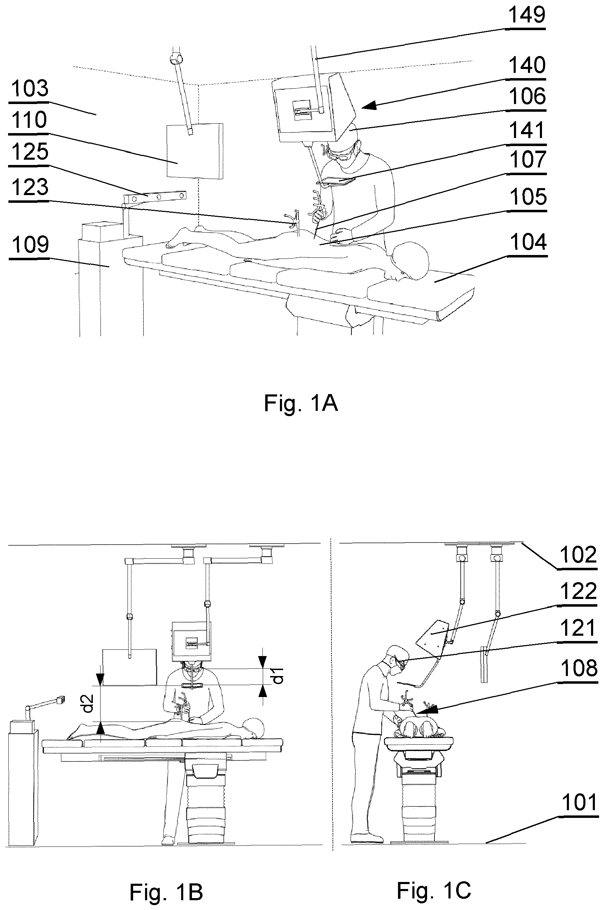

The system presented herein is comprises a 3D display system 140 to be implemented directly on real surgical applications in a surgical room as shown in Figs. 1A-1C. The 3D display system 140 comprises a 3D display 142 for emitting a surgical navigation image 142A towards a see-through mirror 141 that is partially transparent and partially reflective, such that an augmented reality image 141A collocated with the patient anatomy in the surgical field 108 underneath the see-through mirror 141 is visible to a viewer looking from above the see-through mirror 141 towards the surgical field 108.

The surgical room typically comprises a floor 101 on which an operating table 104 is positioned. A patient 105 lies on the operating table 104 while being operated by a surgeon 106 with the use of various surgical instruments 107. The surgical navigation system as described in details below can have its components, in particular the 3D display system 140, mounted to a ceiling 102, or alternatively to the floor 101 or a side wall 103 of the operating room. Furthermore, the components, in particular the 3D display system 140, can be mounted to an adjustable and/or movable floor-supported structure (such as a tripod). Components other than the 3D display system 140, such as the surgical image generator 131, can be implemented in a dedicated computing device 109, such as a stand-alone PC computer, which may have its own input controllers and display(s) 110.

In general, the system is designed for use in such a configuration wherein the distance d1 between the surgeon's eyes and the see-through mirror 141, is shorter than the distance d2, between the see-through mirror 141 and the operative field at the patient anatomy 105 being operated.

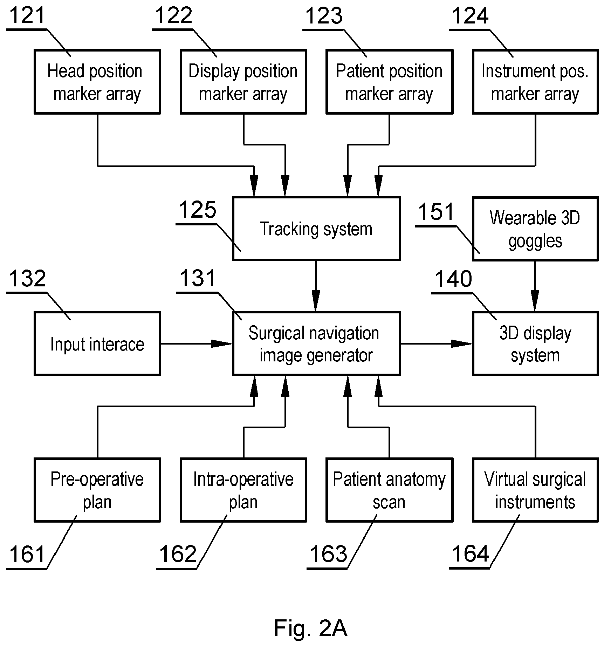

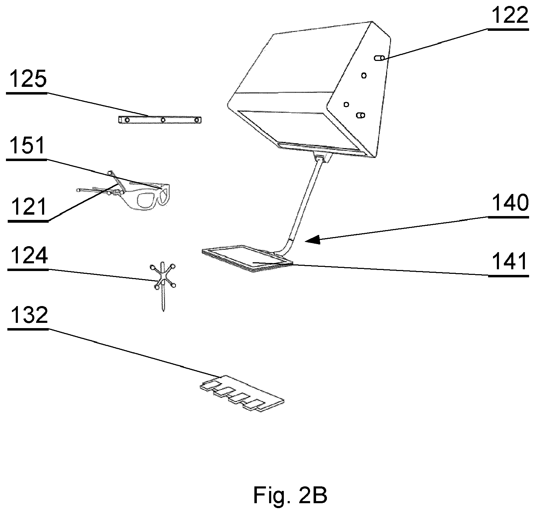

Fig. 2A shows a functional schematic presenting connections between the components of the surgical navigation system and Fig. 2B shows examples of physical embodiments of various components.

The surgical navigation system comprises a tracking system for tracking in real time the position and/or orientation of various entities to provide current position and/or orientation data. For example, the system may comprise a plurality of arranged fiducial markers, which are trackable by a fiducial marker tracker 125. Any known type of tracking system can be used, for example in case of a marker tracking system, 4-point marker arrays are tracked by a threecamera sensor to provide movement along six degrees of freedom. A head position marker array 121 can be attached to the surgeon's head for tracking of the position and orientation of the surgeon and the direction of gaze of the surgeon - for example, the head position marker array 121 can be integrated with the wearable 3D glasses 151 or can be attached to a strip worn over surgeon's head.

A display marker array 122 can be attached to the see-through mirror 141 of the 3D display system 140 for tracking its position and orientation, as the see-through mirror 141 is movable and can be placed according to the current needs of the operative setup.

A patient anatomy marker array 123 can be attached at a particular position and orientation of the anatomy of the patient.

A surgical instrument marker array 124 can be attached to the instrument whose position and orientation shall be tracked.

Preferably, the markers in at least one of the marker arrays 121-124 are not coplanar, which helps to improve the accuracy of the tracking system.

Therefore, the tracking system comprises means for real-time tracking of the position and orientation of at least one of: a surgeon's head 106, a 3D display 142, a patient anatomy 105, and surgical instruments 107. Preferably, all of these elements are tracked.

A surgical navigation image generator 131 is configured to generate an image to be viewed via the see-through mirror 141 of the 3D display system. It generates a surgical navigation image 142A comprising data of at least one of: the pre-operative plan 161 (which are generated and stored in a database before the operation), data of the intra-operative plan 162 (which can be generated live during the operation), data of the patient anatomy scan 163 (which can be generated before the operation or live during the operation) and virtual images 164 of surgical instruments used during the operation (which are stored as 3D models in a database).

The surgical navigation image generator 131, as well as other components of the system, can be controlled by a user (i.e. a surgeon or support staff) by one or more user interfaces 132, such as foot-operable pedals (which are convenient to be operated by the surgeon), a keyboard, a mouse, a joystick, a button, a switch, an audio interface (such as a microphone), a gesture interface, a gaze detecting interface etc. The input interface(s) are for inputting instructions and/or commands.

All system components are controlled by one or more computer which is controlled by an operating system and one or more software applications. The computer may be equipped with a suitable memory which may store computer program or programs executed by the computer in order to execute steps of the methods utilized in the system. Computer programs are preferably stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein. The computer(s) can be placed within the operating room or outside the operating room. Communication between the computers and the components of the system may be performed by wire or wirelessly, according to known communication means.

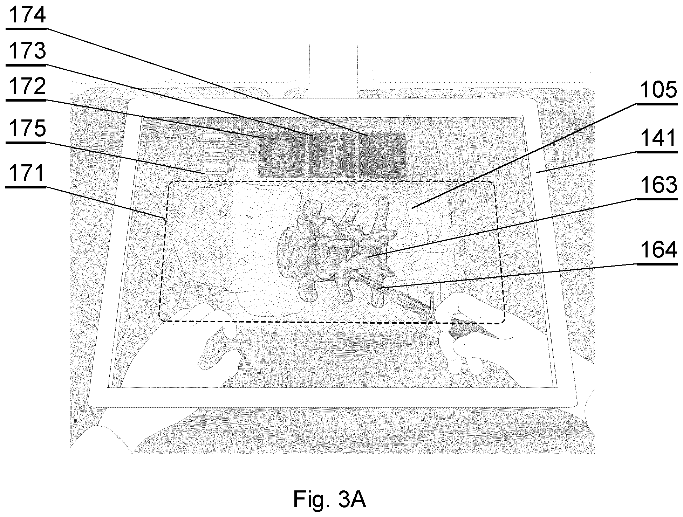

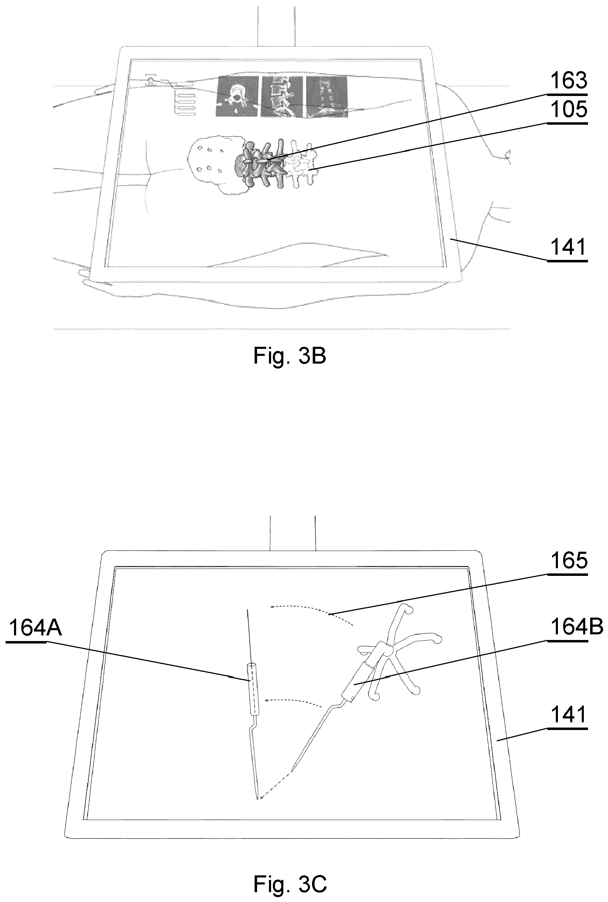

The aim of the system is to generate, via the see-through mirror 141, an augmented reality image such as shown in examples of Figs. 3A-3E. When the surgeon looks via the see-through mirror 141, the surgeon sees the augmented reality image 141A which comprises:
- the real world image: the patient anatomy, surgeon's hands and the instrument currently in use (which may be partially inserted into the patient's body and hidden under the skin);
- and a computer-generated surgical navigation image 142A comprising:
   o a 3D image 171 representing at least one of: the virtual image of the patient anatomy 163, the virtual image of the instrument 164 or surgical guidance indicating ideal trajectory and placement of surgical instruments 107, according to the pre-operative plans 161 (as shown in Fig. 3C);
   ∘ preferably, three different orthogonal planes of the patient anatomy data 163: coronal 174, sagittal 173, axial 172;
   o preferably, a menu 175 for controlling the system operation.

If the 3D display 142 is stereoscopic, the surgeon shall use a pair of 3D glasses 151 to view the augmented reality image 141A. However, if the 3D display 142 is autostereoscopic, it may be not necessary for the surgeon to use the 3D glasses 151 to view the augmented reality image 141A.

Preferably, the images of the orthogonal planes 172, 173, 174 are displayed in an area next (preferably, above) to the area of the 3D image 171, as shown in Fig. 3A, wherein the 3D image 171 occupies more than 50% of the area of the see-through mirror 141.

The location of the images of the orthogonal planes 172, 173, 174 may be adjusted in real time depending on the location of the 3D image 171, when the surgeon changes the position of the head during operation, such as not to interfere with the 3D image 171.

The surgical navigation image 142A is generated by the image generator 131 in accordance with the tracking data provided by the fiducial marker tracker 125, in order to superimpose the anatomy images and the instrument images exactly over the real objects, in accordance with the position and orientation of the surgeon's head. The markers are tracked in real time and the image is generated in real time. Therefore, the surgical navigation image generator 131 provides graphics rendering of the virtual objects (patient anatomy, surgical plan and instruments) collocated to the real objects according to the perspective of the surgeon's perspective.

For example, surgical guidance may relate to suggestions (virtual guidance clues 164) for placement of a pedicle screw in spine surgery or the ideal orientation of an acetabular component in hip arthroplasty surgery. These suggestions may take a form of animations that show the surgeon whether the placement is correct. The suggestions may be displayed both on the 3D holographic display and the orthogonal planes. The surgeon may use the system to plan these orientations before or during the surgical procedure.

In particular, the 3D image 171 is adapted in real time to the position and orientation of the surgeon's head. The display of the different orthogonal planes 172, 173, 174 may be adapted according to the current position and orientation of the surgical instruments used.

Fig. 3B shows an example indicating collocation of the virtual image of the patient anatomy 163 and the real anatomy 105.

For example, as shown in Fig. 3C, the 3D image 171 may demonstrate a mismatch between a supposed position of the instrument according to the pre-operative plan 161, displayed as a first virtual image of the instrument 164A located at its supposed position, and an actual position of the instrument, visible either as the real instrument via the see-through mirror and/or a second virtual image of the instrument 164B overlaid on the current position of the instrument. Additionally, graphical guiding cues, such as arrows 165 indicating the direction of the supposed change of position, can be displayed.

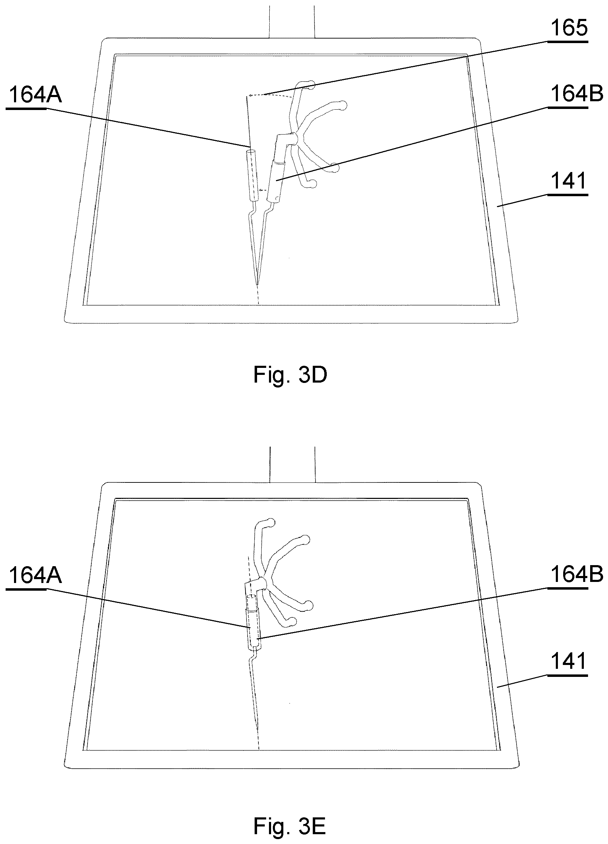

Fig. 3D shows a situation wherein the tip of the supposed position of the instrument displayed as the first virtual image 164A according to the pre-operative plan 161 matches the tip of the real surgical instrument visible or displayed as the second virtual image 164B. However, the remainder of objects do not match, therefore the graphical cues 165 still indicate the need to change its position. The surgical instrument is close to the correct position and the system may provide information on how close the surgical instrument is to the planned position.

Fig. 3E shows a situation wherein the supposed position of the real surgical instrument matches the position of the instrument according to the pre-operative plan 161, i.e. the correct position for surgery. In this situation the graphical cues 165 are no longer displayed, but the virtual images 164A, 164B may be changed to indicate the correct position, e.g. by highlighting it or blinking.

The see-through mirror (also called a half-silvered mirror) 141 is at least partially transparent and partially reflective, such that the viewer can see the real world behind the screen but the screen also reflects the surgical navigation image generated by the display apparatus located above it.

For example, a see-through mirror as commonly used in teleprompters can be used. For example, the see-through mirror 141 can have a reflective and transparent rate of 50R/50T, but other rates can be used as well.

The surgical navigation image is emitted from above the see-through mirror 141 by the 3D display 142.

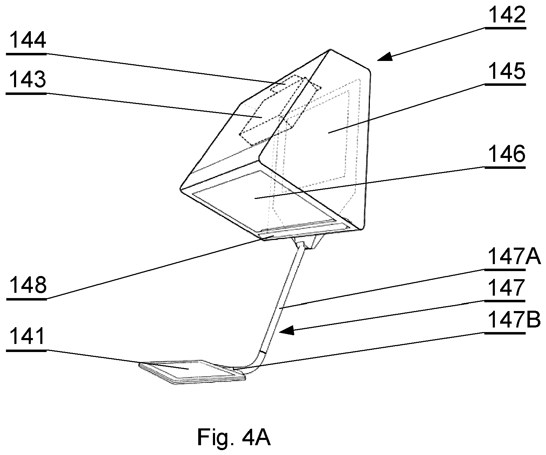

In an example embodiment as shown in Fig. 4A, a special design of the 3D display 142 is provided that is compact in size to facilitate its mounting within a limited space at the operating room. That design allows generating images of relatively large size, taking into account the small distance between the 3D display 142 and the see-through mirror 141, without the need to use wide-angle lens that could distort the image.

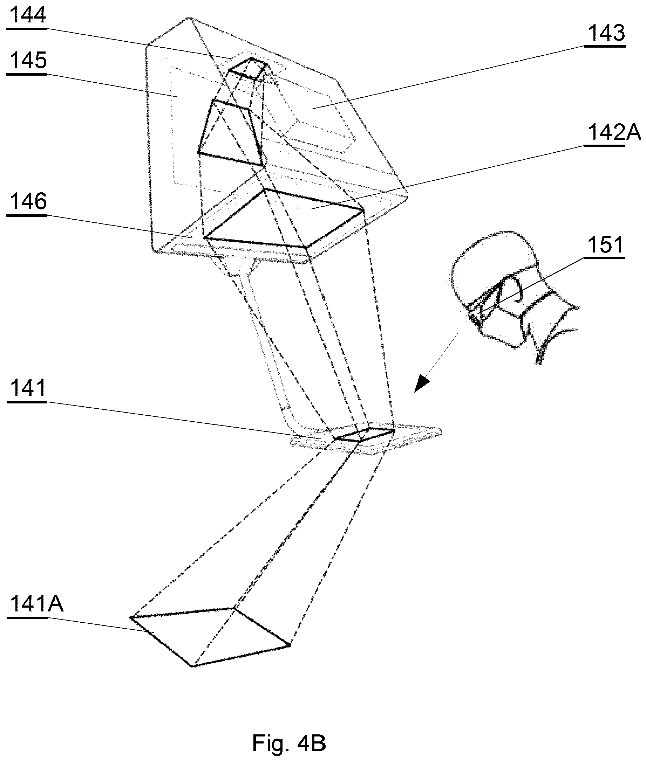

The 3D display 142 comprises a 3D projector 143, such as a DLP projector, that is configured to generate an image, as shown in Fig. 4B (by the dashed lines showing image projection and solid lines showing images generated on particular reflective planes). The image from the 3D projector 143 is firstly refracted by an opaque top mirror 144, then it is refracted by an opaque vertical mirror 145 and subsequently placed on the correct dimensions on a projection screen 146 (which can be simply a glass panel). The projection screen 146 works as a rearprojection screen or a small bright 3D display. The image displayed at the projection screen 146 is reflected by the see-through mirror 141 which works as an augmented reality visor. Such configuration of the mirrors 144, 145 allows the image generated by the 3D projector 143 to be shown with an appropriate size at the projection screen 146. The fact that the projection screen 146 emits an enlarged image generated by the 3D projector 143 makes the emitted surgical navigation image bright, and therefore well visible when reflected at the see-through mirror 141. Reference 141A indicates the augmented reality image as perceived by the surgeon when looking at the see-through mirror 141.

Alternatively, a projector of a wider angle than the source projector of Fig. 4A can be used as the 3D projector 143, wherein the wide angle projector is emitting the surgical navigation image directly towards the projection screen 146 of the embodiment described in Fig. 4A.

Alternatively, a 3D monitor can be used directly in place of the projection screen 146.

The see-through mirror 141 is held at a predefined position with respect to the 3D projector 143, in particular with respect to the 3D projector 143, by an arm 147.

In one embodiment, as shown in Fig. 4A, the arm 147 may have a first portion 147A fixed to the casing of the 3D display 142 and a second portion 147B detachably fixed to the first portion 147A. The first portion 147A may have a protective sleeve overlaid on it. The second portion 147B, together with the see-through mirror 141, may be disposable in order to keep sterility of the operating room, as it is relatively close to the operating field and may be contaminated during the operation. The arm can also be foldable upwards to leave free space of the work space when the arm and augmented reality are not needed

If a need arises to adapt the position of the augmented reality screen with respect to the surgeon's head (for example, to accommodate the position depending on the height of the particular surgeon), the position of the whole 3D display system 140 can be changed, for example by manipulating an adjustable holder (a surgical boom) 149 on Fig 1A, by which the 3D display 142 is attachable to an operating room structure, such as a ceiling, a wall or a floor.

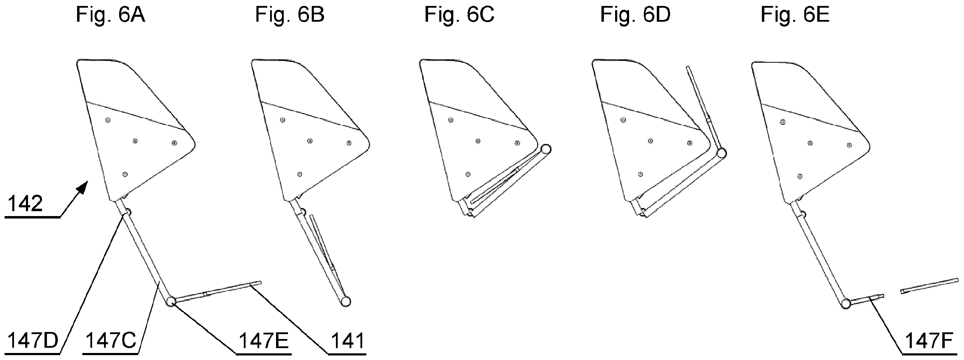

In another embodiment, as shown in Figs. 6A-6E, the arm 147 may comprise a bar 147C attached at a first end via a first pivot joint 147D to the body of the 3D display and having a second end with a second pivot joint 147E to which the see-through mirror 141 is attached. Therefore, the see-through mirror 141 can be arranged in various positions that do not obstruct the field of view of the surgeon, such as shown in Figs. 6B, 6C and 6D. As further shown in Fig. 6E, the see-through mirror can be detachably attached to a bar 147F attached to the second pivot joint 147E.

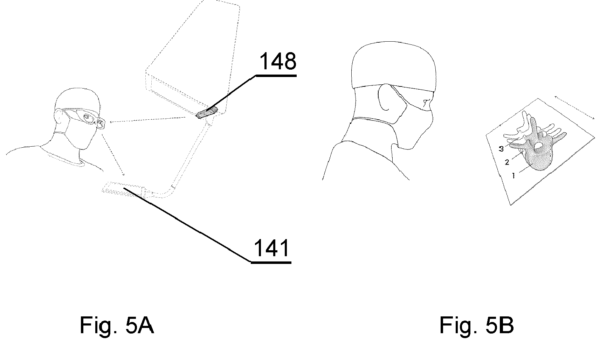

An eye tracker 148 module can be installed at the casing of the 3D display 142 or at the see-through mirror 141 or at the wearable glasses 151, to track the position and orientation of the eyes of the surgeon and input that as commands via the gaze input interface to control the display parameters at the surgical navigation image generator 131, for example to activate different functions based on the location that is being looked at, as shown in Figs. 5A and 5B.

For example, the eye tracker 148 may use infrared light to illuminate the eyes of the user without affecting the visibility of the user, wherein the reflection and refraction of the patterns on the eyes are utilized to determine the gaze vector (i.e. the direction at which the eye is pointing out). The gaze vector along with the position and orientation of the user's head is used to interact with the graphical user interface. However, other eye tracking algorithms techniques can be used as well.

It is particularly useful to use the eye tracker 148 along with the pedals 132 as the input interface, wherein the surgeon may navigate the system by moving a cursor by eye sight and inputting commands (such as select or cancel) by pedals.

## Claims

1. A surgical navigation system, comprising:
a 3D display (142); and
a see-through mirror (141) that is partially transparent and partially reflective and coupled to the 3D display (142) by an arm (147),
the 3D display configured to receive, from a surgical navigation image generator (131), a surgical navigation image (142A) comprising at least the patient anatomy data (163), in accordance with current position and orientation data based on real-time tracking of a surgeon's head (106), the 3D display (142), and a patient anatomy (105),
the 3D display (142) further configured to emit and refract the surgical navigation image (142A) towards the see-through mirror (141), such that an augmented reality image (141A) collocated with the patient anatomy in the surgical field (108) underneath the see-through mirror (141) is visible to a viewer looking from above the see-through mirror (141) toward the surgical field (108).

2. The system of claim 1, wherein the 3D display (142) comprises a 3D projector (143), a first opaque mirror (144), a second opaque mirror (145) and a projection screen (146), wherein the 3D projector (143) is configured to project the surgical navigation image towards the first opaque mirror (144), the first opaque mirror is configured to refract the surgical navigation image towards the second opaque mirror (145), and the second opaque mirror is configured to refract the surgical navigation image towards the projection screen (146), the projection screen (146) further refracting the surgical navigation image (142A) towards the see-through mirror (141).

3. The system of claim 2, wherein the first opaque mirror is a top mirror that is oriented horizontally and the second opaque mirror is side mirror oriented vertically in the 3D display (142).

4. The system of claim 1, wherein the 3D display (142) comprises a 3D projector (143), a first opaque mirror (144), a second opaque mirror (145) and a projection screen (146), wherein the first opaque mirror (144), the second opaque mirror (145) and the projection screen (146) are positioned such that the surgical navigation image emitted towards the see-through mirror (141) is cast on the see-through mirror (141) at a predefined dimension in accordance to the current position and orientation data provided by the tracking system (125).

5. The system of any of previous claims, wherein the see-through mirror (141) is configured to be positioned, when the system is in use, at a distance (dl) from the surgeon's head which is shorter than the distance (d2) from the surgical field (108) of the patient anatomy (105).

6. The system of any of previous claims, wherein the surgical navigation image (142A) is a first surgical navigation image (142A) and at least one of the 3D display (142) and the see-through mirror (141) is configured to be arranged in a set of positions such that an adjustment of the at least one of the 3D display (142) and the see-through mirror (141) from a first position from the set of positions to a second position from the set of positions causes the 3D display to receive from the surgical navigation image generator (131) a second surgical navigation image (142A) comprising at least the patient anatomy data (163), in accordance to the current position and orientation data, the current position and orientation data including the adjustment of the at least one of the 3D display (142) and the see-through mirror (141) to the second position from the set of positions.

7. The system of any of previous claims, wherein the arm (147) is at least one of a detachable arm (147B) or a foldable arm (147C-147F).

8. The system of any of previous claims, wherein the see-through mirror (141) is configured to be fifty percent reflective and fifty percent transmissive (50R/50T)

9. The system of any of previous claims, wherein the surgical navigation image generator (131) is controllable by an input interface (132) comprising at least one of: foot-operable pedals (132), a microphone, a joystick, and an eye-tracker (148).

10. The system of any of previous claims, further comprising an adjustable ceiling-mounted surgical boom (149), wherein the 3D display (142) is attached to the adjustable ceiling-mounted surgical boom (149).

11. The system of any of previous claims, further comprising an adjustable floor-supported structure, wherein the 3D display (142) is attached to the adjustable floor-supported structure.

12. The system of any of previous claims, wherein the tracking system (125) comprises plurality of arranged fiducial markers, including a head array (121), a display array (122), a patient anatomy array (123), a surgical instrument array (124), as well as a fiducial marker tracker (125), which is configured to determine in real time the position and orientation of the surgeon's head, the 3D display (142).

13. The system of claim 12, wherein at least one of the head array (121), the display array (122), the patient anatomy array (123), and the instrument array (124) contains several fiducial markers that are not all coplanar.

14. The system of any of previous claims, wherein the 3D display (142) is further configured to receive from the surgical navigation image generator (131) the surgical navigation image (142A) including a virtual image (164) of one or more virtual surgical instruments (164), the virtual image (164) of the one or more surgical instruments being based on data from a tracking system (125) configured to track current position and orientation data of the one or more surgical instruments.

15. A method for providing an augmented reality image during an operation, comprising:
providing a 3D display (142) with a see-through mirror (141) that is partially transparent and partially reflective, the see-through mirror (141) being fixed to the 3D display (142) by an arm (147) and held at a predefined position with respect to the 3D display (142) by the arm (147);
receiving, via the 3D display and from a surgical navigation image generator (131), a surgical navigation image (142A) comprising a patient anatomy data (163), in accordance to a current position and orientation data provided by a tracking system (125), the current position and orientation data being based on real-time tracking of a surgeon's head (106), the 3D display (142), and a patient anatomy (105); and
emitting and refracting, via the 3D display the surgical navigation image (142A) towards a see-through mirror (141) that is partially transparent and partially reflective, such that an augmented reality image (141A) collocated with the patient anatomy in the surgical field (108) underneath the see-through mirror (141) is visible to a viewer looking from above the see-through mirror (141) towards the surgical field (108).
